**Europäisches Patentamt**

**European Patent Office**

**Office européen des brevets**

(11) Publication number: **0 110 333**
**B1**

(12) **EUROPEAN PATENT SPECIFICATION**

(45) Date of publication of patent specification: **24.06.87**

(21) Application number: **83111793.2**

(22) Date of filing: **27.11.80**

(60) Publication number of the earlier application in accordance with Art. 76 EPC: **0 030 014**

(51) Int. Cl.⁴: **A 61 B 1/26**

(54) **Improved laryngoscope.**

(30) Priority: **28.11.79 US 98271**
**28.04.80 US 144704**

(43) Date of publication of application:
**13.06.84 Bulletin 84/24**

(45) Publication of the grant of the patent:
**24.06.87 Bulletin 87/26**

(84) Designated Contracting States:
**CH DE FR GB LI NL**

(56) References cited:
**US-A-3 766 909**
**US-A-3 826 248**
**US-A-4 153 356**

(73) Proprietor: **Upsher, Michael S.**
**2957 Adeline Drive**
**Burlingame California 94010 (US)**

(72) Inventor: **Upsher, Michael S.**
**2957 Adeline Drive**
**Burlingame California 94010 (US)**

(74) Representative: **Dipl.-Phys.Dr. Manitz Dipl.-Ing., Dipl.-Wirtsch. Finsterwald Dipl.-Chem.Dr. Heyn Dipl.-Phys. Rotermund Morgan, B.Sc.(Phys.) Robert-Koch-Strasse 1 D-8000 München 22 (DE)**

EP 0 110 333 B1

## Description

The present invention relates to a laryngoscope comprising: a handle including a first light source and power source means; and a first blade carrying a light guide but no light source and having means for mounting the first blade with said handle in a way which causes said power source means to energise the first light source while placing said light guide in optical communication with said first light source. Furthermore, the invention relates to a laryngoscope handle per se and to a laryngoscope blade therefor.

A laryngoscope of the initially named kind is shown in DE—B—27 38 202. Various other laryngoscopes are disclosed in US—A—2,646,036, US—A—3,986,854 and US—A—4,086,919. An endoscope which is related to a laryngoscope is disclosed in US—A—3,896,793.

The principal object underlying the present invention is to provide an improved laryngoscope handle having a large light bulb therein which can be a light source for a laryngoscope blade with a fibre optic light guide, yet the handle can also be used with a conventional laryngoscope blade incorporating its own light source.

In order to satisfy this object there is provided, in accordance with the present invention, a laryngoscope of the initially named kind which is characterised by the provision of a second blade including a second light source thereon; and by means forming part of said handle and part of said second blade for mounting the second blade with said handle in a way which causes said power source means to energise said second light source without energising said first light source.

Advantageous developments of this laryngoscope are set forth in the accompanying subordinate claims 2 to 6. The invention also relates to a laryngoscope handle as defined in claims 7 to 9 and to a laryngoscope blade as defined in claim 10.

The invention will now be explained in more detail with reference to the accompanying drawings in which

Fig. 1 is a view showing a base for a laryngoscope blade;

Fig. 2 is an enlarged fragmentary cross-sectional view of the bulb in the base of Fig. 1;

Figs. 3 and 4 are views similar to Fig. 1 but showing additional ways of mounting the bulb in the base;

Fig. 5 is an enlarged, fragmentary cross-sectional view of a laryngoscope handle having a bulb mounted therein for a standard laryngoscope blade or a blade having a fiber-optics bundle for transmitting light to the outer end of the blade from the light bulb in the handle.

Fig. 6 is a view of the handle of Fig. 5 but rotated through an angle of 90°;

Fig. 7 is a top plan view of the handle of Fig. 5;

Fig. 8 is a view similar to Fig. 5 but showing the bulb and a switch in a cartridge for mounting in a conventional laryngoscope handle;

Fig. 9 is a view of the cartridge of Fig. 8 but rotated through an angle of 90°;

Fig. 10 is an exploded view of the handle showing the cartridge for insertion thereinto;

Fig. 11 is a cross-sectional view of a metallic adapter for mounting in a standard laryngoscope handle;

Fig. 12 is a second cross-sectional view of the metallic adapter in Fig. 11;

Fig. 13 is an enlarged cross-sectional view of the locking mechanism for holding the adapter of Figs. 11 and 12 in place of a handle;

Figs. 14, 15 and 16 are different elevational views of the adapter;

Fig. 17 is a top plan view of the adapter;

Figs. 18 and 19 are views similar to Figs. 11 and 12 but showing a plastic adapter;

Fig. 20 is a side elevational view of either adapter on a conventional handle;

Fig. 21 is a top plan view of the adapter of Figs. 18 and 19;

Fig. 22 is a side elevational view, partly in section, of a handle having a bulb mounted in the upper end thereof for use with a conventional laryngoscope blade;

Fig. 23 is a view similar to Fig. 22 but showing a light bulb mounted in a central cartridge in the handle;

Fig. 24 is a view similar to Fig. 23 but showing the blade pivoted out of position, disabling the light bulb in the cartridge;

Fig. 25 is a side elevational view of the cartridge of Fig. 24;

Fig. 26 is a top plan view of the cartridge of Fig. 25;

Fig. 27 is another type of blade with a handle with a light bulb in a central cartridge;

Fig. 28 is a bottom plan view of a portion of the blade for use with the handle of Fig. 27;

Fig. 29 is a front elevational view of the blade of Fig. 28;

Fig. 30 is another type of central cartridge in a handle for use with a conventional blade;

Fig. 31 is a view similar to Fig. 30 but showing the central cartridge of Fig. 30 with a non-conventional blade;

Figs. 32 and 33 are views similar to Figs. 30 and 31 but show additional embodiments of a central cartridge with conventional and non-conventional blades;

Fig. 34 is a cross-sectional view of the cartridge of Fig. 30;

Fig. 35 is a cross-sectional view of the cartridge of Fig. 32;

Fig. 36 is a top plan view of the cartridge of Fig. 35;

Fig. 37 is a view of another central cartridge for use with conventional and non-conventional blades;

Fig. 38 is a top plan view of the cartridge of the handle of Fig. 37;

Fig. 39 is a fragmentary side elevational view of the base of a non-conventional blade for use with the cartridge of Fig. 37;

Fig. 40 is a view similar to Fig. 5 but showing a light bulb in a side portion of the handle;

Fig. 41 is another view of the handle of Fig. 40;

Fig. 42 is a top plan view of the handle of Fig. 40;

Figs. 43 and 44 are side and bottom views, respectively, of the base of the non-conventional blade for use with the handle of Fig. 40;

Fig. 45 is a fragmentary view of the handle of Fig. 40 showing how the light bulb is energized.

Fig. 46 is a view similar to Fig. 40 but showing another cartridge in the handle; and,

Fig. 47 is a view showing slight modification of the handle of Fig. 46.

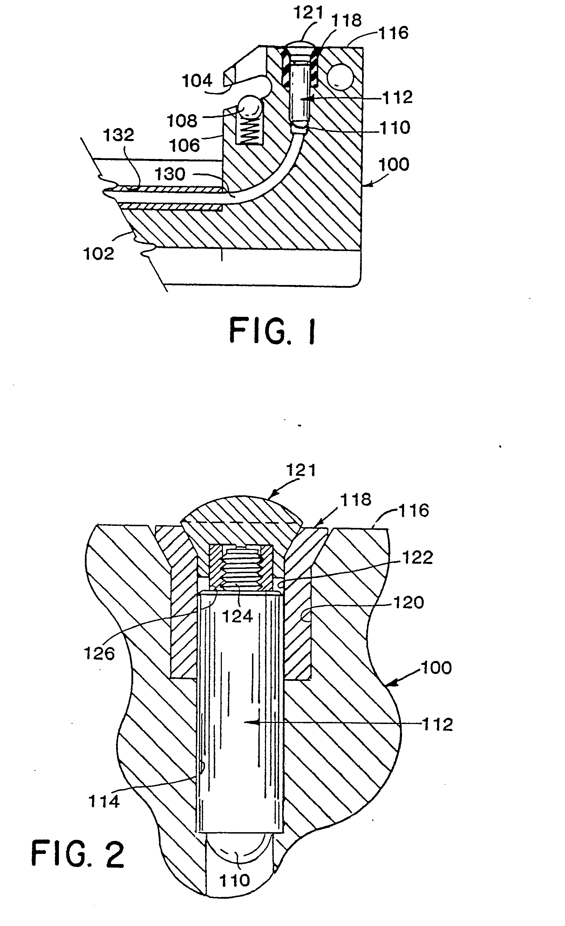

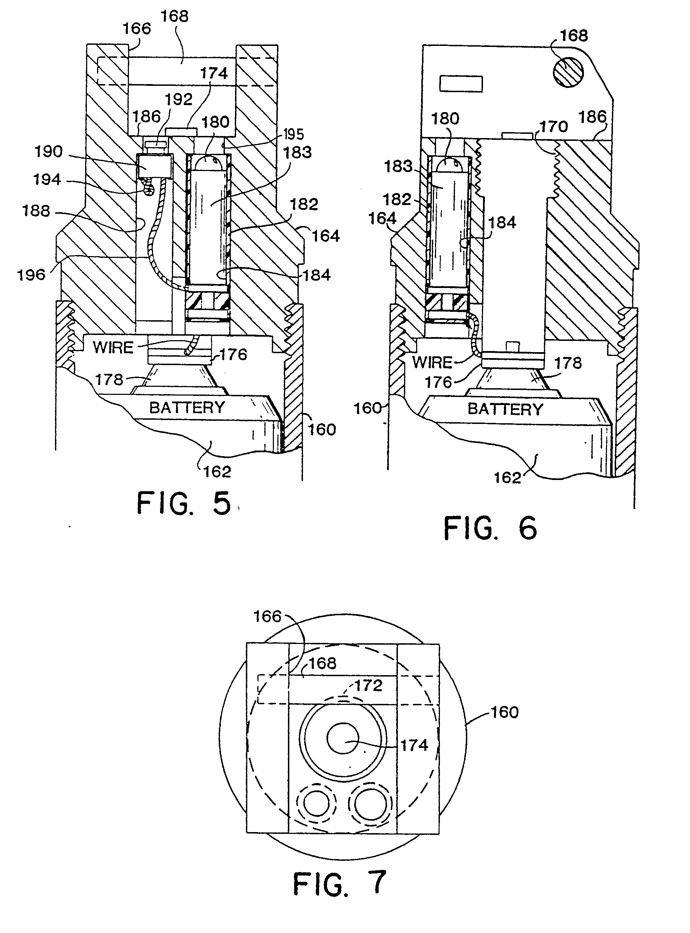

Fig. 1 is a view showing an embodiment 100 of a base for a laryngoscope blade 102. The blade of Fig. 1 can be used with the laryngoscope handle of Fig. 5. A slot 104 for receiving the crossbar on a standard handle extends into face 106 of base 100. A ball detent 108 is spring biased into slot 104 so that the ball releasably holds the blade in place on the bar of an associated handle.

A light bulb 110 is received in a cylindrical housing 112 (Fig. 2) in a passage 114 extending into the base from end face 116 thereof. An insulator 118 is press fitted into a second passage 120 and holds housing 112 in place in passage 114. An electrical contact 121 is press fitted into the central bore 122 of insulator 118 and makes electrical contact through a threaded projection 124 extending into a second tubular insulator 126 between the annular extension 128 of contact 121 and threaded end 124. Contact 121 is to be coupled electrically to the corresponding electrical contact on a standard handle when the handle is coupled to base 100 in the usual fashion. This causes bulb 110 to be energized to direct light through a fiber optic bundle 130 (Fig. 1) which extends through base 100 and along a passage 132 in blade 102.

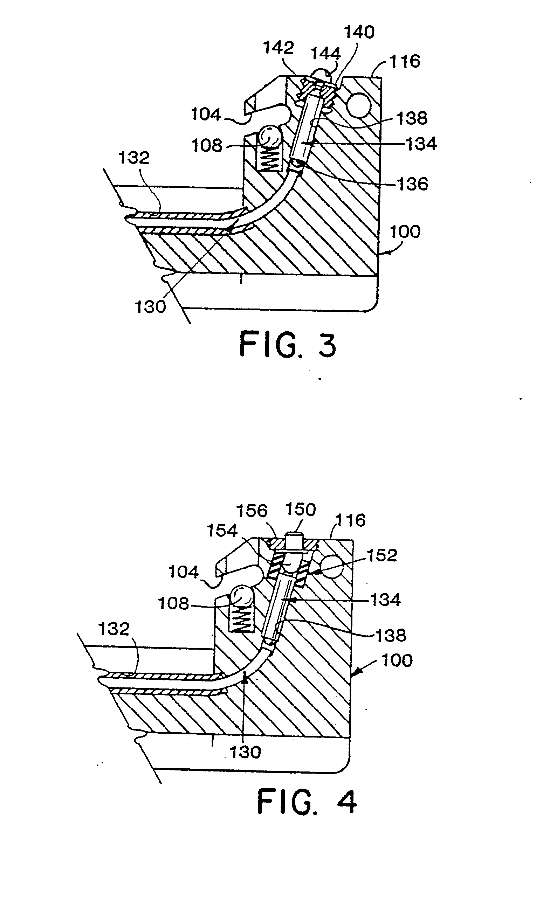

Preferred embodiments of blade base 100 are shown in Figs. 3 and 4 in which another type of housing 134 having a bulb 136 extends in an inclined passage 138 in base 100. An insulator 140 surrounds base screw 142 making contact with the center top pin of bulb 136 and screw 142 has a head 144 defining the end contact for the bulb. This head projects outwardly from end face 116 of base 100. The other electrical connection is through the case of housing 134 and base 100.

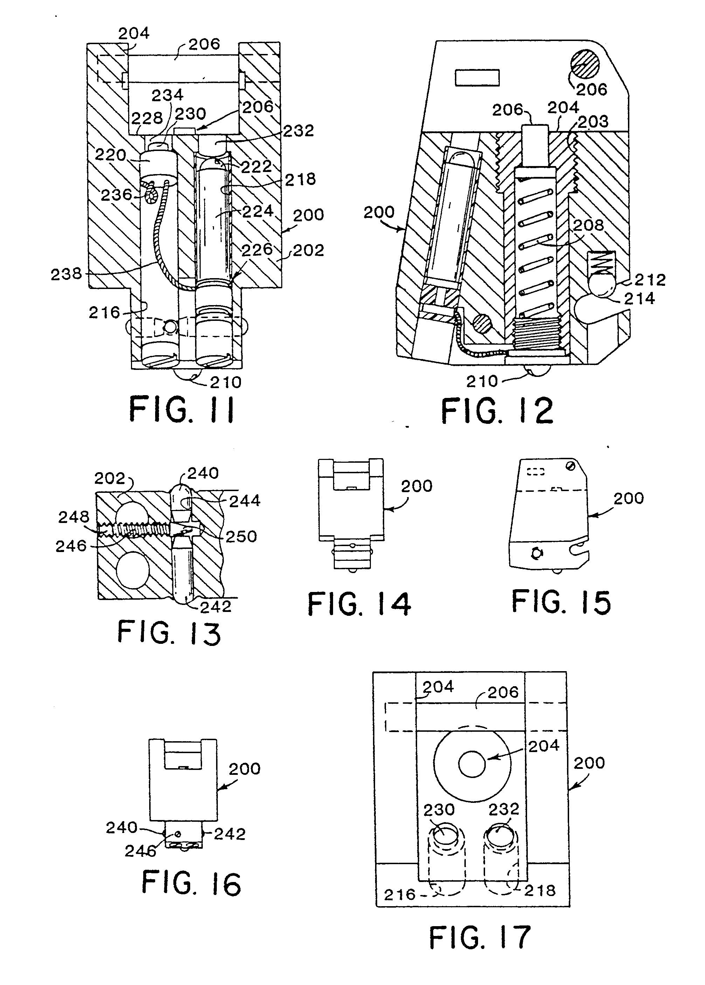

Fig. 4 shows that housing 134 can have an electrical contact 150 which projects straight out from end face 116 rather than at an angle as shown in Fig. 3. A spacing grommet 152 receives one end 154 of contact 150, the opposite end of the contact being at an angle with relative to end 154 as shown in Fig. 12. Contact 150 passes through an insulator 156 threaded into the end of face 116 of base 100. In both embodiments of base 100 as shown in Figs. 3 and 4, fiber optics bundle 130 mates easily with passage 138.

Fig. 5 shows a laryngoscope handle 160 containing a battery 162 and provided with a threaded end member 164 having a slot 166 at the upper end thereof. A crossbar 168 at end 166 is adapted to be inserted into a usual slot, such as slot 104 of base 100 (Fig. 1) for connecting the base to the handle. However, in the case of handle 160, member 164 has a space 170 for a cartridge normally found in a standard laryngoscope handle, such as a standard cartridge 172 of the type shown in Figs. 133 and 134. The cartridge has a contact 174 at its upper end which, through a spring (not shown) inside the housing of the cartridge, makes electrical connection with an end contact 176 which, as shown in Fig. 5, engages the end terminal 178 of battery 162.

Member 164 has a bulb 180 in a cylindrical housing 183 and an insulator 182 disposed within a passage 184 extending inwardly from the end face 186 of member 164. A second passage 188 parallel with passage 184, contains a microswitch 190 having a pushbutton actuator 192 recessed inwardly from end face 186. Also, bulb 180 is spaced inwardly from end face 186. Wires 194 and 196 make electrical connection between battery 162, a light bulb 180 and microswitch 190 when the microswitch is actuated as its actuator 192 is forced inwardly. Handle 160 is to be used with either a first "non-conventional" blade of the type having a base provided with a fiber optics bundle, which is insertable within the opening 198 forming a part of passage 184, or with a second "conventional" blade having its own light bulb. The non-conventional blade base also has a projection for insertion into the upper open space above actuator 192 of microswitch 190 so that when the projection is forced into this opening, actuator 192 is depressed is closes switch 190 to connect battery 162 and light bulb 180. Then, light will be transmitted through the fiber optics bundle to the outer end of the laryngoscope blade. If a conventional blade is used, voltage from the battery 162 is supplied to the light bulb at the outer end of the blade through standard cartridge 172 in the usual manner.

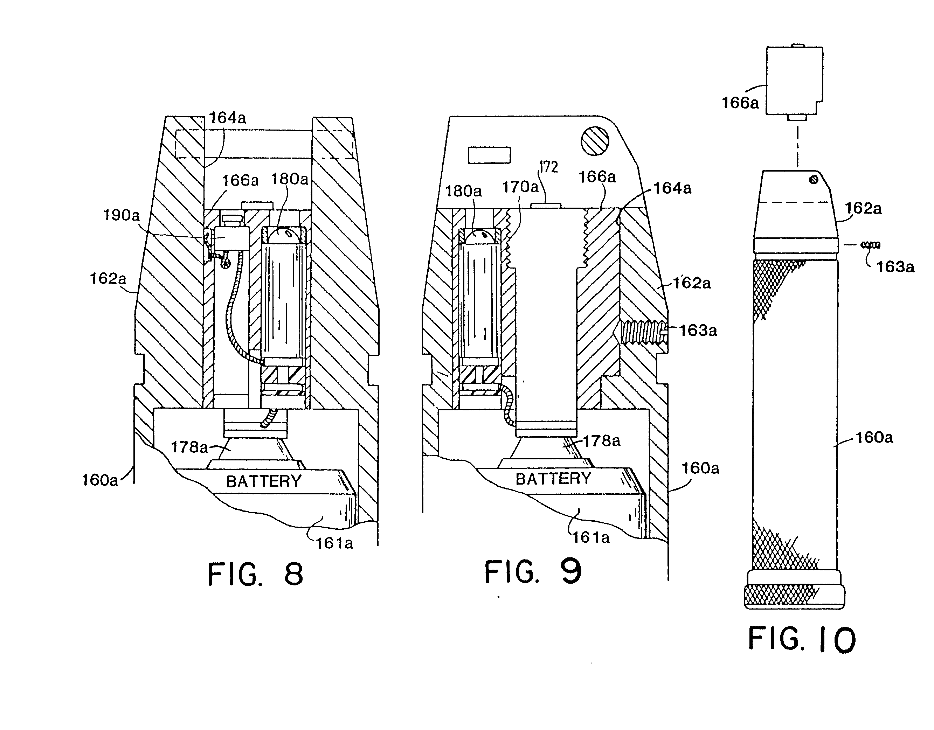

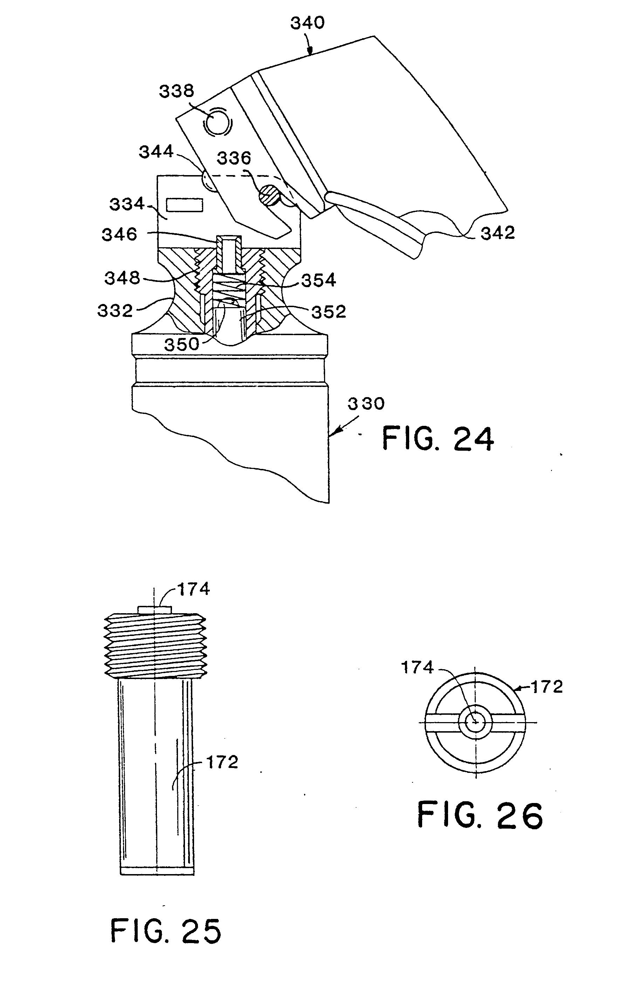

Figs. 8 and 9 are views showing a handle 160a which has an upper member 162a provided with a central, rectangular slot 164a for receiving a cartridge 166a containing a bulb 180a, a standard cartridge 172 of the type shown in Fig. 25 and 26 in a space 170a, and a microswitch 190a. The cartridge is inserted into opening 164a until the cartridge makes electrical contact with the end terminal 178a of a battery 161a. The cartridge is held in place by a set screw 163a threaded into a side opening 165a in member 162a. The cartridge 166a and handle 160a are shown in elevation in Fig. 10.

Figs. 11 and 12 show several views of a metallic adapter for use on a handle with a conventional or non-conventional laryngoscope blade. The adapter, denoted by the numeral 200, includes a metallic member 202 having the usual slot 204 provided with a crossbar 206 for attachment to the base of a blade having a slot, such as slot 104 of base 100 (Fig. 1). Member 202 has a central passage 203 for receiving a conventional cartridge 204 of the type having an electrical contact 206 coupled through a metallic, coil spring 208 to an end contact 210 for connection to a battery contact in the conventional handle with which adapter 200 is to be used. Adapter 200 has a side slot 212 and a ball detent 214 to receive the conventional crossbar of a conventional laryngoscope handle in the usual manner.

Member 202 has two additional, side-by-side, generally parallel passages 216 and 218, passage 216 having a microswitch 220 near the upper end thereof and passage 218 having a light bulb 222 in a housing 224 surrounded by an insulating sleeve 226 inserted into passage 218. The light bulb is recessed inwardly from the upper end surface 228 of member 202. Similarly, the pushbutton actuater 230 of microswitch 220 is also recessed below surface 228. Thus, the recess 232 aligned with the light bulb is adapted to receive the end of the fiber optics bundle on the base of a non-conventional blade which is to be coupled to the upper end of adapter 200. Similarly, the recess 234 aligned with the microswitch is adapted to receive a projection on the nonconventional blade base for actuating the microswitch, which through wires 236 and 238, connects light bulb with the battery in the handle with which adapter 200 is associated. In this way, light will travel from the light bulb through the fiber optics bundle to the outer end of the blade for illuminating the throat of the patient as the blade is inserted into the throat. If a conventional blade is used with adapter 200, light bulb 222 is not actuated and cartridge 204 will connect the light bulb on the conventional blade with the standard cartridge and battery of the handle.

Figs. 14, 15 and 16 show elevational views of adapter 200. Fig. 14 shows a top plan view of the adapter.

The adapter is held in place by a structure shown in Fig. 13. To this end, a pair of wedges 240 and 242 are mounted in a passage 244 in body 202 as shown in Figs. 11, 12 and 13. A set screw 246 is threaded into a passage 248 perpendicular to passage 244 and has a conical end face 250 which bears against the adjacent ends of wedges 240 and 242 to force the wedges outwardly and against the adjacent surfaces of the conventional handle with which adapter is associated. Fig. 16 also shows screw 246 and the way in which wedges 240 and 242 project laterally from a base portion 252 of adapter 200.

Passages 216 and 218 of adapter 200 are located at an angle so that they can be carried by adapter 200 without interference with cartridge 204. If they were parallel with the cartridge, there would not be sufficient structure to allow for the presence of the light bulb and the microswitch without weakening the overall structure of the adapter.

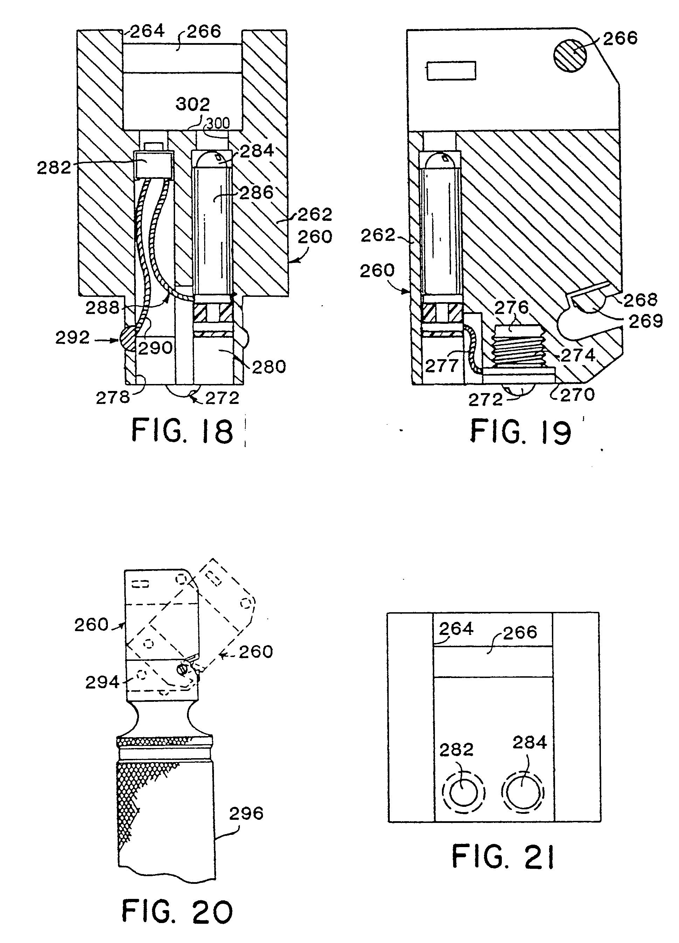

Figs. 18—21 show another form of an adapter which is made of plastic rather than metal as in the case of adapter 200. Adapter 260 has a plastic body 262 provided with an upper slot 264 an a crossbar 266 for attachment to the base of a conventional or non-conventional laryngoscope blade. Body 262 also has a slot 268, a detent 269, and a bottom surface 270 provided with an electrical contact 272 for attachment in the usual fashion with the upper end of a conventional laryngoscope handle. This is shown in Fig. 20, the adapter 260 being shown in an operative position in full lines and in a position ready to be removed from the handle in dashed lines.

Contact 272 has a threaded upper end 274 which is threaded into a passage 276 extending inwardly from surface 270 of body 262. A wire 277 makes electrical contact between a light bulb 284 and contact 272. The adapter has a pair of side-by-side, generally parallel passages 278 and 280 for receiving, respectively, a microswitch 282 and light bulb 284, the latter being carried in a housing 286 and coupled by wire 288 to microswitch 282. A second wire 290 from the microswitch extends to a metal contact 292 for making electrical contact with the adjacent metallic part 294 of conventional handle 296 (Fig. 20). Thus, when adapter 260 is placed on handle 296 in the manner shown in Fig. 20, bulb 284 will be ready to be energized because contact 272 will be coupled to the central terminal of the battery in handle 296 and contact 292 will make electrical contact with part 294 (Fig. 20). When switch 282 is closed, i.e., when the push-button actuator 298 of the switch is pressed downwardly by a projection on a non-conventional blade base coupled with the upper end of the adapter, the light will come on and will pass through a fiber optics bundle whose end is inserted in the recessed opening 300 of passage 280 adjacent to surface 302 of the adapter body 262. Adapter 260 shows that the light source 284 can be accommodated without enlarging the adapter body as in the case of the adapter shown in Figs. 11 and 12.

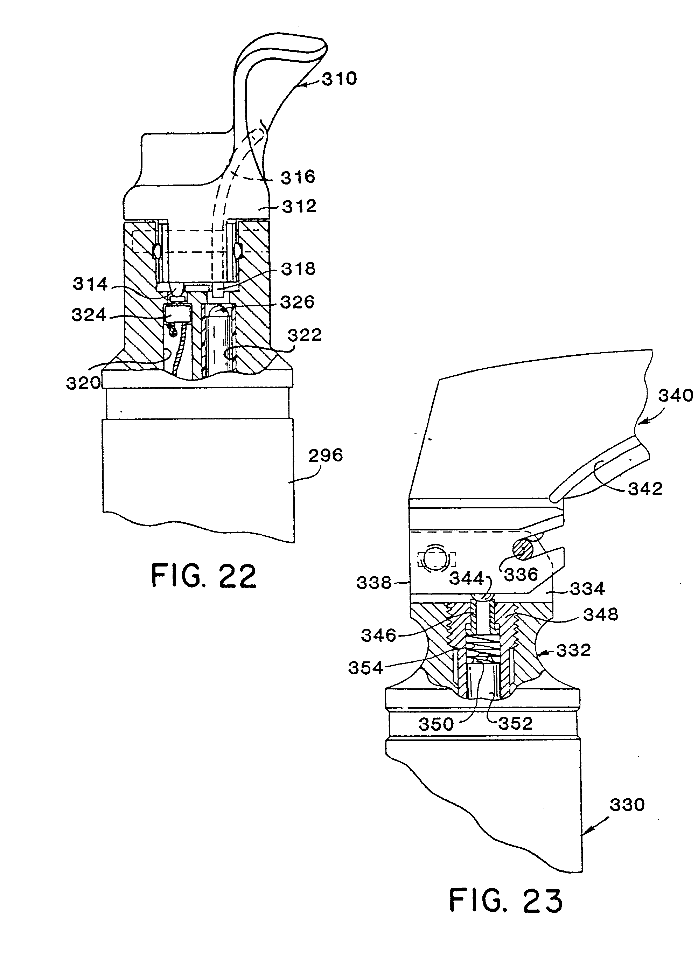

Fig. 22 is a view of a non-conventional laryngoscope blade 310 having a base 312 provided with a projection 314 and a fiber optics bundle 316 having an end 318. Projection 314 and 318 are shown inserted into the end recesses of a pair of passages 320 and 322 containing respectively a microswitch 324 and a light bulb 326. Thus, projection 314 is shown as actuating the microswitch 324 while the fiber optics end 318 is shown aligned optically with the light bulb 326 to receive light therefrom. Fig. 22 illustrates how a base of a blade is used to actuate the light source of adapter 200 of Figs. 11 and 12 and adapter of 260 of Figs. 18 and 19.

Another type of handle for a conventional or non-conventional blade is denoted by the numeral 330 and is shown in Figs. 23 and 24. Handle 330 has an upper end 332 provided with a slotted part 334 and a crossbar 336 for receiving the base 338 of a conventional blade 340. The blade has a wiring channel 342 which extends to the outer end of the blade for connection to a light bulb but has one end which is electrically coupled to a metallic projection 344 projecting outwardly from the end face of base 338.

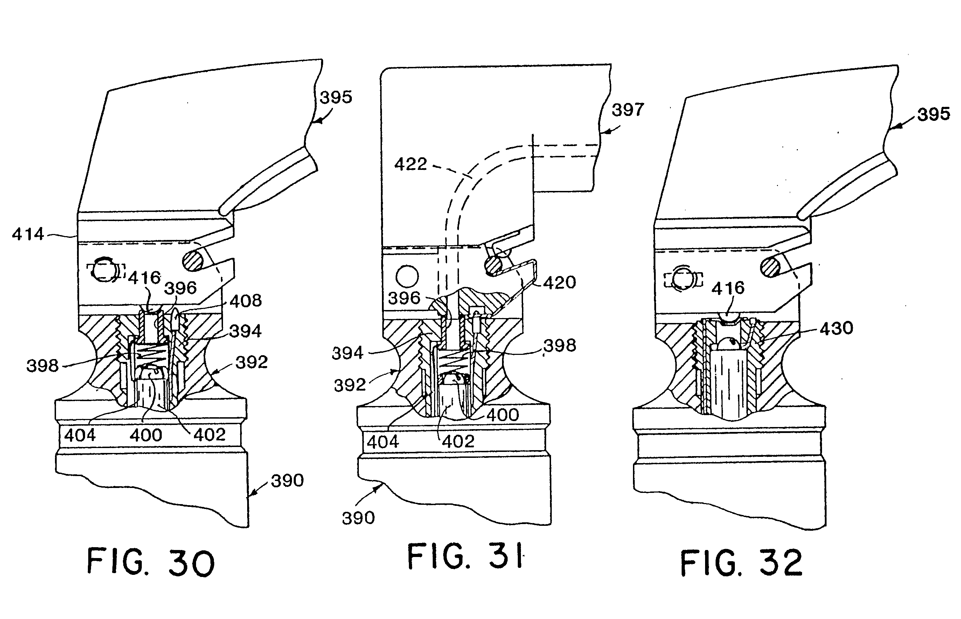

End 332 has a cartridge 348 threadably coupled thereto, the cartridge containing a light bulb 350 in a housing 352 which makes electrical contact with a terminal of a battery (not shown) carried by handle 330. The body of cartridge 348 is of insulating material so that it will not interfere with the electrical connection made to the battery by housing 352. A coil spring 354 is in the space above the light bulb and makes electrical contact with projection 344 through a tubular metallic sleeve 346 slidable in the upper end of the passage which contains light bulb 350. Before blade base 338 is snapped into place as shown in Fig. 24, sleeve 346 projects outwardly and does not make electrical contact with base 338. When the base is in place as shown in Fig. 31, sleeve 346 places housing 352 in electrical contact with projection 344 through spring 354.

Thus, voltage from the battery will be applied through the cartridge, through the tubular sleeve 346, and through the wiring in channel 342 to the light bulb at the outer end of the blade. Light bulb 350 will not be actuated in this case.

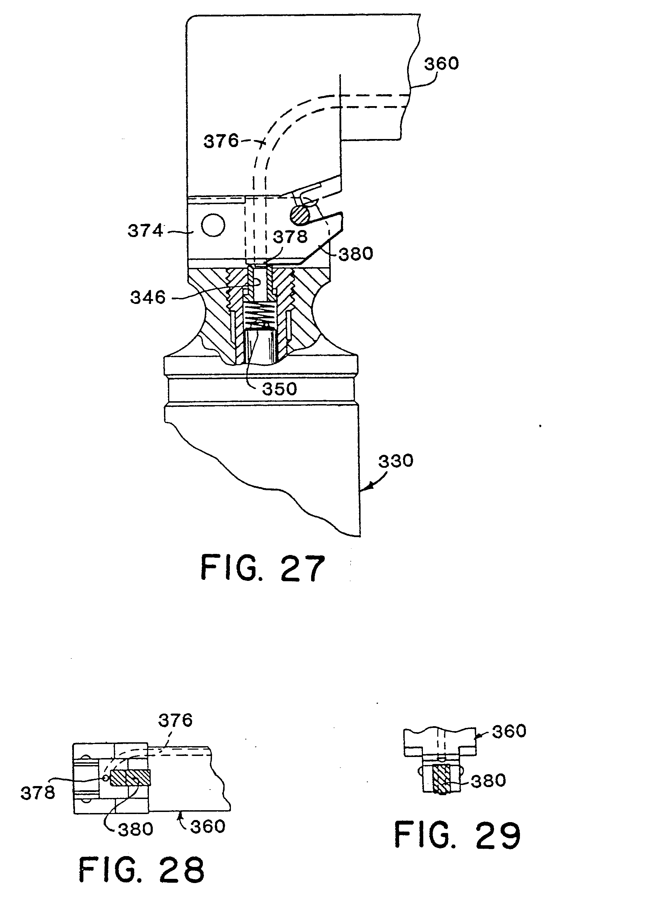

Fig. 27 shows a non-conventional blade 360 with handle 330. Blade base 374 has a fiber optics bundle 376 having one end 378 alignable with sleeve 346 when the blade base 374 is in place as shown in Fig. 27. When this occurs, a conductive metal tape 380 on the bottom surfaces of blade base 374 makes electrical contact between sleeve 346 and the crossbar 336 of handle 330. In this way, an electrical circuit between the light bulb 350 and the battery in handle 330 is completed to turn the light bulb on and cause light to travel upwardly through sleeve 346 and into and through the fiber optics light guide 376 to the outer end of the blade. Figs. 28 and 29 show different views of the blade base. These views also show the position of metallic strip or tape 380 with respect to the adjacent end 378 of the fiber optics bundle 376.

Figs. 30 and 31 show a handle 390 having an upper end 392 provided with a different embodiment of a cartridge 394 for use either with a conventional blade 395 (Fig. 30) or with a non-conventional blade 397 (Fig. 31). The cartridge has an upper tubular sleeve 396, coil spring 398, a light bulb 400 and a housing 402 and a first wire 404 connecting metallic sleeve 396 with one part of the battery in handle 392 when a conventional blade is used. A second wire 406 having an upper contact 408 is electrically connected to housing 402 whereby the other part of the battery in the handle can be connected to the bulb when a non-conventional blade is used.

Blade 395 has a base 414 provided with a metallic projection 416 which makes electrical contact with sleeve 396 when the blade is in place as shown in Fig. 30. This causes the light bulb at the outer end of the blade to be energized by the electrical circuit comprised of the metal of the handle itself and wire 404.

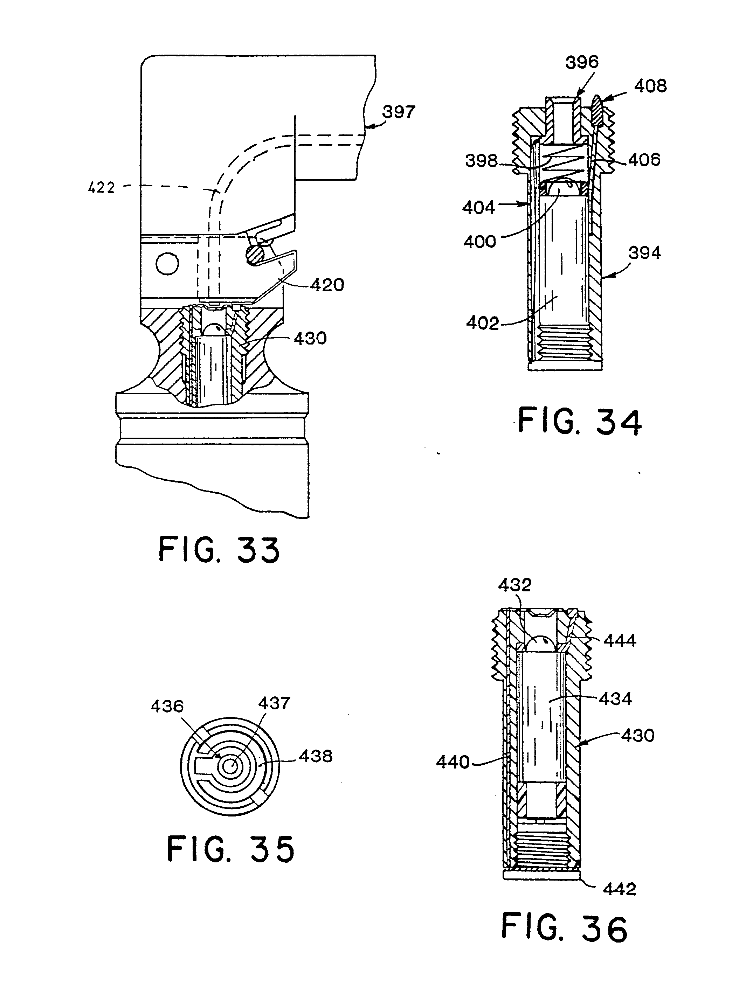

In the case of non-conventional blade 397, the base of the blade has a metallic conductive strip or tape 420 which makes electrical contact with contact tip 408 in a manner shown in Fig. 31 to actuage light bulb 400. A fiber optics light bundle 422 extends into sleeve 396, so that when the light bulb 400 is energized, light will enter the fiber optics light bundle and be passed outwardly to the outer end of the blade 397. Fig. 34 shows the cartridge 394 in greater detail.

Fig. 35 shows a different embodiment of the cartridge denoted by the numeral 430 for use with handle 390. It includes a light bulb 432 in a housing 434 having a first upper electrical contact 436 surrounded by a second electrical contact 438 as shown in Fig. 36. A center hole 437 is in contact 436. A wire 440 makes electrical connection between contact 436 and a bottom contact 442 which engages the battery central terminal in the handle with which cartridge 430 is used. A second wire 444 makes electrical connection with contact 438 and is also connected to housing 434.

Cartridge 430 in handle 390 can be used with conventional blade 395 in the manner shown in Fig. 32. In this case, contact 416 engages contact 436 to connect to the battery through wire 440 while contact 438 is not used. When blade is in the operating position of Fig. 31, light bulb 432 is not energized but battery voltage is applied to the light bulb at the outer end of the blade 410.

When cartridge 430 is used with non-conventional blade 397, contact 438 make electrical connection with tape 420 as shown in Fig. 32 and this causes bulb 432 to be actuated to in turn cause light to pass through central hole 437 (Fig. 36) in contact 436 and then into the fiber optics bundle 432. The light then travels to the outer end of the fiber optics bundle at the outer end of blade 397.

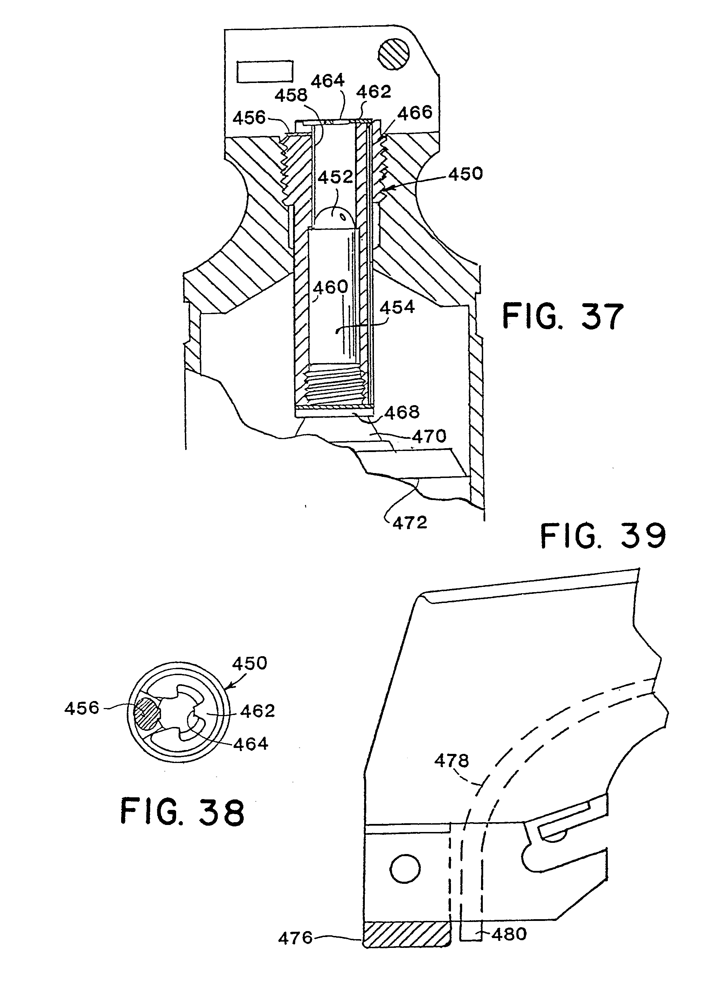

Fig. 37 shows another embodiment of a cartridge 450 for use either with a conventional blade or a non-conventional blade similar to blades 395 and 397 of Figs. 30—33. Cartridge 450 is mounted in a handle 390 and has a light bulb 452 in a housing 454. A first metallic element 456 near the upper end of the cartridge has a lead or wire 458 coupled to housing 454 at location 460. A second metallic member 462 has a central recess 464 therein. Contact 462 makes electrical contact by a wire 466 to the bottom contact 468 on the cartridge which makes electrical contact with the central contact 470 of a battery 472 in the handle 390 with which the cartridge is used.

When a conventional blade is used, the metallic projection of the blade, such as projection 416 on blade 397 of Fig. 30, will make electrical contact with contact 462 which, through wire 466 and end contact 468 will make electrical connection with battery terminal 470 and in bypassing relationship to light bulb 452. Thus, the light bulb 452 will not be energized but voltage will be applied to the light bulb on the outer end of the conventional blade connected to the handle 474.

When a non-conventional blade of the type shown in Fig. 39 is used, the blade will have a deformable metallic projection 476 which will engage element 456, causing bulb 452 to be energized to cause light to travel upwardly and through a fiber optics light bundle 478 whose lower end 480 will extend into recess 464 optically aligned with bulb 452.

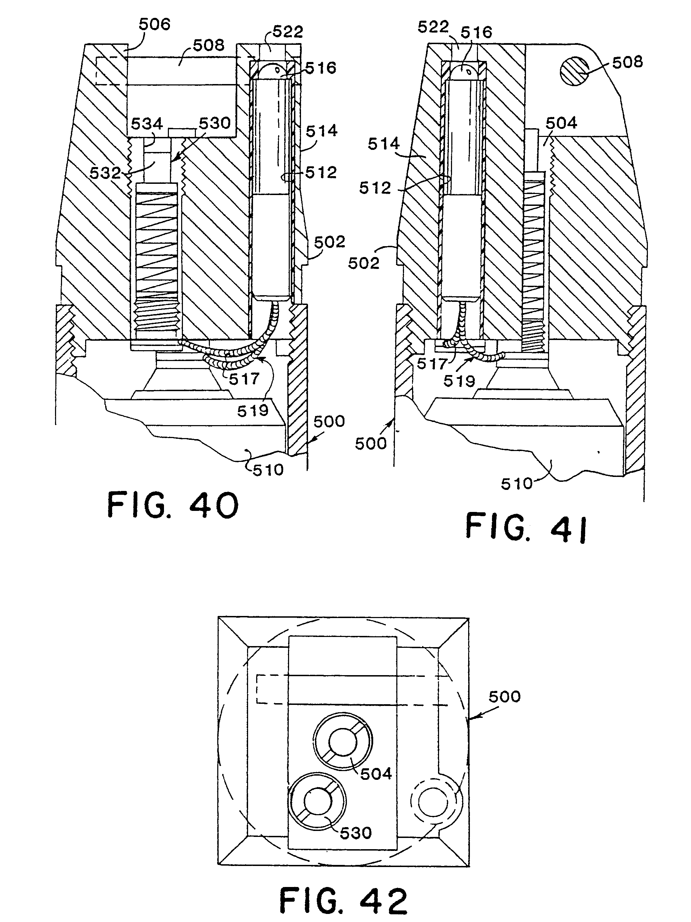

Figs. 40—44 show another type of handle 500 which can be used either with a conventional or non-conventional laryngoscope blade. In this embodiment, handle 500 has an upper member 502 threadably mounted on the handle body as shown in Figs. 40 and 41. Member 502 has a conventional cartridge 504 of the type shown in Figs. 25 and 26 for use with a conventional blade having a light source on the outer end of the blade. When the blade base is coupled in the usual fashion to slot 506 and with the crossbar 508 of member 502, voltage is supplied from battery 510 in handle 500 through the blade base and the wiring channel to the light bulb is at the outer end of the blade.

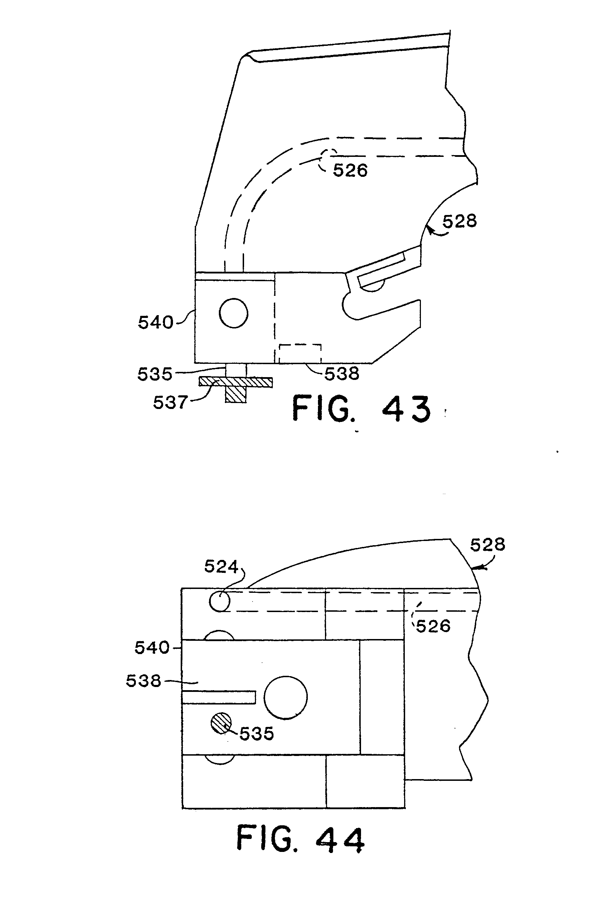

For use with a non-conventional blade, member 502 has a passage 512 near one side 514 thereof for receiving a light bulb 516 in a housing 518 surrounded by an insulating sleeve 520. Bulb 516 is recessed to present an opening 522 for receiving one end 524 (Fig. 44) of a fiber optics light bundle 526 of a non-conventional blade 528 (Figs. 43 and 44).

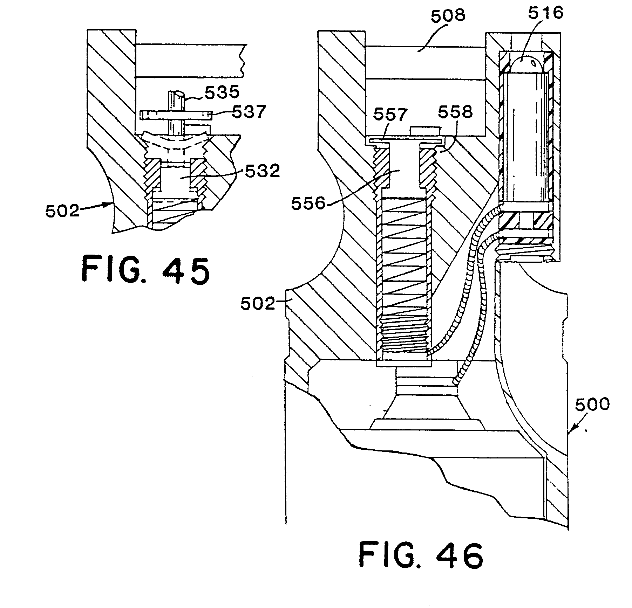

A second cartridge 530 similar in all respects to cartridge 504 is in a passage which is offset from the center line of member 502 as shown in Fig. 42. Cartridge 530 has a member 532 which is shiftable in a passage 534 but which can be engaged by a metallic shaft 535 (Fig. 45) carried by a non-conventional laryngoscope blade (Fig. 43). Shaft 535 has a flexible metallic washer 537 which makes electrical contact with handle 502 as shown in dashed lines to cause light bulb 516 to be supplied with voltage through wires 517 and 519 from battery 510 when shaft 535 on the bottom of face 538 of blade base 540 of blade 528 extends into recess 534. Thus, light from light bulb 516 will enter end face 524 of the fiber optics light guide 526 and will travel to the outer end of blade 528. In this way handle 500 can accommodate a conventional blade and a non-conventional disposable blade.

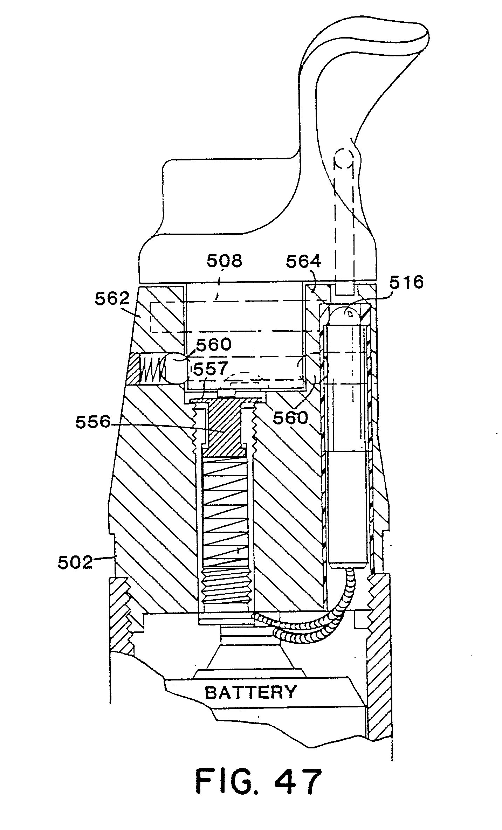

Figs. 46 and 47 show modifications of the cartridge of Figs. 40—45 wherein the cartridge has a reciprocal member 556 with a flat top part 557 which is forced downwardly by projection on a non-conventional blade to make electrical contact with the base of the blade and the adjacent part 558 of the blade. Fig. 47 shows spring biased ball detents 560 carried by the handle parts 562 and 564 for entering recesses in the opposite sides of blade base 566 to releasably hold the base to the handle.

**Claims**

1. A laryngoscope comprising: a handle (160; 160a; 296; 330; 390; 500; 502) including a first light source (180; 180a; 222; 284; 326; 350; 400; 432; 452; 516) and power source means (162; 161a; 472; 516); and a first blade (310; 360; 397; 528) carrying a light guide (316; 376; 422; 478; 526) but no light source and having means for mounting the first blade with said handle in a way which causes said power source means (162; 161a; 472; 510) to energise the first light source (180; 180a; 222; 284; 326; 350; 400; 432; 452; 516) while placing said first light guide in optical communication with said first light source, characterised by the provision of a second blade (102; 340; 395) including a second light source (110; 136) thereon; and by means forming part of said handle and part of said second blade for mounting the second blade (102; 340; 395) with said handle (160, 160a; 296; 330; 390; 500; 502) in a way which causes said power source means (162; 161a; 472; 510) to energise said second light source (110; 136) without energising said first light source (180; 180a; 222; 284; 326; 350; 400; 432; 452; 516).

2. A laryngoscope in accordance with claim 1 wherein said handle has an uppermost blade connecting end having first contact means (192; 190a, 230; 282; 324; 346; 408; 438; 456; 532; 557) electrically connected to said power source means (162; 161a; 472; 510); wherein said first blade has means for mounting it with the blade connecting end of said handle in a way which engages said first contact means (192; 190a; 230; 282; 324; 346; 408; 438; 456; 532; 557) for causing said power source means (162; 161a; 472; 510) to energise said light source (180; 180a; 222; 284; 326; 350; 400; 432; 452; 516) carried by said handle, wherein second contact means (312; 540) is provided at said uppermost blade connecting end, said second contact means being electrically isolated from said first contact means and also connected to said power source means; and wherein said second blade (102; 340; 395) has means for mounting it with the blade connecting end of said handle in a way which engages said second contact means without engaging said first contact means whereby to cause said power source means to energise said second light source.

3. A laryngoscope in accordance with claim 1, wherein said first blade (310; 528) has a forwardmost end and a rearwardmost end, the latter defining a mounting base (312; 540); wherein said means for mounting the first blade with said handle (296; 502) in a way which causes said power source means (510) to energise the first said light source comprises a first cooperating arrangement forming part of said handle and part of said base, said cooperating arrangement including a passage (320) in said handle, and electrical connection means (324; 532) for coupling said light source with said power source, said electrical connection means being disposed within said passage; wherein said electrical connection means does not extend outwardly of said passage; wherein said arrangement also includes a projecting member (314; 537) carried by the base of said first blade for engaging said electrical connection means when said first blade is disengagably connected with said handle in order to cause said power source means to energise said first light source, and wherein said handle (296; 502) includes a second cooperating arrangement (504) separate from said last-mentioned arrangement for cooperating with said second blade such that when said second blade is disengagably connectable with said handle said second arrangement causes the power source means (510) in said handle (296; 502) to actuate the light source carried by said second blade, while not causing said power source means to actuate the light source (326; 537) carried by said handle.

4. A laryngoscope in accordance with any one of the preceding claims wherein the light source (222; 284) included in said handle is provided in an adapter (200; 260) mounted on a basic handle for coupling said first and second blades to said basic handle, wherein said basic handle has a first pair of parallel spaced apart walls defining a first slot and a first cross-bar extending across said first slot; wherein said basic handle is adapted to receive battery means defining said power source means, wherein said adapter has a first end connectable with said basic handle and a second end connectable with the bases of said first and second blades; wherein said first end has a projection having two spaced sidewalls adapted to engage in said first slot, a transverse groove (212; 268) adapted to engage with said first cross-bar, and locking means (240; 242) for locking said projection in position in said first slot; wherein said second end has a second pair of parallel spaced apart walls defining a second slot and a second cross-bar (206; 266) extending across said second slot; wherein the bases of said first and second blades each have a second projection having two spaced sidewalls adapted to engage in said second slot and a transverse groove adapted to engage with said second cross-bar (206; 266), there being detent means for releasably securing said bases with said second end of said adapter in an engaged position; wherein said handle has a first electrical contact substantially centrally disposed in said first slot facing a cooperating second contact (210; 272) provided on a base portion of said first projection; wherein said second contact is electrically connected to a third contact (206) substantially centrally disposed in said second slot; wherein said second contact is electrically connected to said light source (222; 284) provided in said adapter; wherein a switch (220; 282) is provided in said adapter, said switch being actuatable by said first blade in said engaged position to complete an electrical circuit between said light source (222; 284) and said battery means; wherein said second blade can be substituted for the first said blade, said second blade having a fourth contact cooperating with said third contact and a fifth contact cooperating with said second cross-bar to complete an electrical circuit to said battery means to energise the light source of said second blade, said second blade being constructed so as not to actuate said switch means; and wherein said first and second cross-bars are electrically connected together and to said battery means when said adapter is locked in a position in said first slot.

5. A laryngoscope in accordance with claim 4, wherein said adapter (200) has a body portion of metal thus ensuring electrical contact between said first and second cross-bars.

6. A laryngoscope in accordance with either of the preceding claims 4 and 5, wherein said locking means (240; 242) comprises a pair of wedges (240, 242) mounted in a passage in said first projection and a set screw (246) for forcing said wedges outwardly against said first pair of spaced apart walls.

7. A laryngoscope handle (160; 160a; 296; 330; 390; 500; 502) for use in a laryngoscope designed to operate with a first blade (310; 360; 397; 528) having a light guide (316; 376; 422; 478; 526), said handle comprising: a hand gripping portion, a handle connecting head portion connected with said hand gripping portion and designed to disengagably connect with said first blade; power source means (162; 161a; 472; 510); a first light source (180; 180a; 222; 284; 326; 350; 400; 432; 452; 516) and means (192; 190a; 230; 282; 324; 346; 408; 438; 456; 532; 557) forming part of said head portion and connected with said power source means and said first light source (180; 180a; 222; 284; 326; 350; 400; 432; 452; 516) for energising the latter when a said first blade is disengagably connected with said head portion, characterised in that the laryngoscope handle is also designed to operate with a second blade (102; 340; 395) having its own second light source (110; 136); and in that means (174; 172; 206; 346; 396; 436; 462; 504) forming part of said head portion and connected with said power source means (162; 161a; 472; 510) is provided for energising a said second light source (110; 136) carried by a said second blade when the latter is disengagably connected with the head portion without causing said first light source (180; 180a; 222; 284; 326; 350; 400; 432; 452; 516) to energise.

8. A laryngoscope handle in accordance with claim 7 in combination with a said first blade (310; 360; 397; 528).

9. A laryngoscope handle in accordance with claim 7 in combination with a said second blade (102; 340; 395).

10. A laryngoscope blade comprising; a blade body (528) having a forwardmost end and a rearwardmost end, the latter defining a mounting base (540) having means for mounting the blade to a cooperating laryngoscope handle (500) such that a specific surface of said base (538) is in confronting relationship with a cooperating surface of said handle; a light guide (526) carried by said blade and extending from its rearwardmost end at said base to its forwardmost end at the forwardmost end of said blade body such that the rearwardmost end of the light guide is placed in optical alignment with a light source (516) carried by said handle (500) when the base of said blade is mounted to said handle; electrical contact means (537) at said base; said contact means being adapted to engage a cooperating contact means (532) on said handle for causing said light source (516) to energise when the base of said blade is disengagably connected to said handle, characterised in that said electrical contact means (537) projects from said specific surface (538) of said base and in that said specific surface of the base of said blade body includes an opening configured to receive a second, projecting contact means forming part of said handle such that the second contact means does not engage the blade when the base of the blade is disengagably connected to the handle.

**Patentansprüche**

1. Laryngoskop mit: einen Handgriff (160; 160a; 296; 330; 390; 500; 502) einschließlich einer ersten Lichtquelle (180; 180a; 222; 284; 326; 350; 400; 432; 452; 516) und Energiequellenmitteln (162; 161a; 472; 516) und einem ersten Spatel (310; 360; 397; 528), der einen Lichtleiter (316; 376; 422; 478; 526), jedoch keine Lichtquelle trägt und Mittel zum Befestigen des ersten Spatels an dem Handgriff in einer Weise besitzt, die das Energiequellenmittel (162; 161a; 472; 510) zum Beaufschlagen der ersten Lichtquelle (180; 180a; 222; 284; 326; 350; 400; 432; 452; 516) veranlaßt, während der Lichtleiter in optische Verbindung mit der ersten Lichtquelle gebracht wird, gekennzeichnet durch das Vorsehen eines zweiten Spatels (102; 340; 395), der eine zweite Lichtquelle (110; 136) daran enthält und durch einen Teil des Handgriffes und einen Teil des zweiten Spatels bildende Mittel zum Befestigen des zweiten Spatels (102; 340; 395) an dem Handgriff (160; 160a; 296; 330; 390; 500; 502) in einer Weise, die das Lichtquellenmittel (162; 161a; 472; 510) zum Beaufschlagen der zweiten Lichtquelle (110; 136) ohne Beaufschlagen der ersten Lichtquelle (180; 180a; 222; 284; 326; 350; 400; 432; 452; 516) veranlaßt.

2. Laryngoskop nach Anspruch 1, bei dem der Handgriff ein oberstes Spatel-Verbindungsende mit ersten Kontaktmitteln (192; 190a; 230; 282; 324; 346; 408; 438; 456; 532; 557) besitzt, die elektrisch mit dem Energiequellenmittel (162; 161a; 472; 510) verbunden sind; wobei der erste Spatel Mittel zu seiner Befestigung an dem Spatelverbindungsende des Handgriffes in einer Weise besitzt, daß Eingriff mit den ersten Kontaktmitteln (192; 190a; 230; 282; 324; 346; 408; 438; 456; 532; 557) erfolgt, um das Energiequellenmittel (162; 161a; 472; 510) zum Beaufschlagen der durch den Handgriff getragenen Lichtquelle (180; 180a; 222; 284; 326; 350; 400; 432; 452; 516) zu veranlassen, wobei zweite Kontaktmittel (312; 540) an dem obersten Spatelverbindungsende vorgesehen sind, die elektrisch isoliert gegen die ersten Kontaktmittel und ebenfalls mit dem Energiequellenmittel verbunden sind; und wobei der zweite Spatel (102; 340; 395) Mittel zu seiner Befestigung an dem Spatelverbindungsende des Handgriffes in einer Weise besitzt, die Eingriff an den zweiten Kontaktmitteln ohne Eingriff an den ersten Kontaktmitteln ergibt, um dadurch das Energiequellenmittel zum Beaufschlagen der zweiten Lichtquelle zu veranlassen.

3. Laryngoskop nach Anspruch 1, bei dem der erste Spatel (310; 528) ein vorderstes Ende und ein hinterstes Ende besitzt, wobei das letztere eine Befestigungsbasis (312; 540) bestimmt; wobei das Mittel zum Befestigen des ersten Spatels an dem Handgriff (296; 502) in einer Weise, die das Energiequellenmittel (510) zum Beaufschlagen der ersten Lichtquelle veranlaßt, eine erste, zusammenwirkende, einen Teil des Handgriffes und einen Teil der Basis bildende Anordnung umfaßt, die einen Durchlaß (320) in dem Handgriff enthält und elektrische Verbindungsmittel (324; 532) zum Kuppeln der Lichtquelle mit der Energiequelle, welche elektrischen Verbindungsmittel innerhalb der Durchlasses untergebracht sind; wobei die elektrischen Verbindungsmittel sich nicht aus dem Durchlaß heraus erstrecken; wobei die Anordnung auch ein durch die Basis des ersten Spatels gehaltenes vorstehendes Teil (314; 537) enthält, um an dem elektrischen Verbindungsmittel anzugreifen, wenn der erste Spatel lösbar mit dem Handgriff verbunden ist, um das Energiequellenmittel zu veranlassen, die erste Lichtquelle zu beaufschlagen, und wobei der Handgriff (296; 502) eine zweite zusammenwirkende Anordnung (504), getrennt von der zuletzt erwähnten Anordnung, enthält zum Zusammenwirken mit dem zweiten Spatel in der Weise, daß, wenn der zweite Spatel lösbar mit dem Handgriff verbindbar is, sie das Energiequellenmittel (510) in dem Handgriff (296; 502) zum Betätigen der durch den zweiten Spatel gehaltenen Lichtquelle veranlaßt, und gleichzeitig das Energiequellenmittel nicht zum Beaufschlagen der durch den Handgriff gehaltenen Lichtquelle (326; 537) veranlaßt.

4. Laryngoskop nach einem der vorangehenden Ansprüche, bei dem die in dem Handgriff enthaltene Lichtquelle (222; 284) in einem Adapter (200; 260) vorgesehen ist, der an einen Handgriff-Basisteil angebracht ist zum Koppeln des ersten und des zweiten Spatels mit dem Handgriff-Basisteil, wobei der Handgriff-Basisteil ein erstes Paar von parallelen, beabstandeten, einen ersten Schlitz definierenden Wänden und eine erste, sich quer zu dem ersten Schlitz erstreckende Querschiene besitzt; wobei das Handgriff-Basisteil zur Aufnahme von die Energiequellenmittel definierenden Batteriemitteln eingerichtet ist, der Adapter ein erstes mit dem Handgriff-Basisteil verbindbares Ende und ein zweites, mit den Basisabschnitten des ersten und des zweiten Spatels verbindbares Ende besitzt; das erste Ende einen Fortsatz mit zwei beabstandeten, zum Eingriff in den ersten Schlitz eingerichteten Seitenwänden, eine zum Eingriff mit der ersten Querschiene eingerichtete Quernut (212; 268) und Sperrmittel (240; 242) zum Versperren des Fortsatzes in seiner Stellung in dem ersten Schlitz besitzt, das zweite Ende ein zweites Paar von parallelen, beabstandeten und einen zweiten Schlitz definierenden Wänden und eine sich quer über den zweiten Schlitz erstreckende zweite Querschiene (206; 266) besitzt, die Basisabschnitte des ersten und des zweiten Spatels jeweils einen zweiten Fortsatz mit zwei beabstandeten, zum Eingriff in den zweiten Schlitz eingerichteten Seitenwänden und eine zum Eingriff mit der zweiten Querschiene (206; 266) eingerichtete Quernut besitzen; Rastmittel zum lösbaren Sichern der Basisteile an dem zweiten Ende des Adapters in einer Eingriffstellung vorgesehen sind; wobei der Handgriff einen im wesentlichen zentral in dem ersten Schlitz angeordneten, einem damit zusammenwirkenden, an einem Grundabschnitt an dem ersten Fortsatz vorgesehenen zweiten Kontakt (210; 272) zugewendeten ersten elektrischen Kontakt besitzt, der elektrisch mit einem im wesentlichen zentral in dem zweiten Schlitz angeordneten dritten Kontakt (206) und elektrisch mit der in dem Adapter vorgesehenen Lichtquelle (222; 284) verbunden ist; ein Schalter (220; 282) an dem Adapter vorgesehen ist, der durch den ersten Spatel in dern Eingriffsstellung betätigbar ist, um einem elektrischen Kreis zwischen der Lichtquelle (222; 284) und dem Batteriemittel zu vervollständigen; der zweite Spatel anstatt des ersten Spatels eingesetzt werden kann, der zeite Spatel einen vierten, mit dem dritten Kontakt zusammenwirkenden Kontakt und einen fünften, mit der zweiten Querschiene zusammenwirkenden Kontakt besitzt, um einen elektrischen Kreis zu dem Batteriemittel zum Beaufschlagen der Lichtquelle des zweite Spatels zu vervollständigen, wobei der zweite Spatel so aufgebaut ist, daß er das Schaltermittel nicht betätigt, und wobei die erste und die zweite Querschiene elektrisch miteinander und mit dem Batteriemittel verbunden sind, wenn der Adapter in einer Stellung im ersten Schlitz versperrt ist.

5. Laryngoskop nach Anspruch 4, bei dem der Adapter (200) einen aus Metall bestehenden Gehäuseabschnitt besitzt und so elektrischen Kontakt zwischen der ersten und der zweiten Querschiene sicherstellt.

6. Laryngoskop nach einem der vorangehenden Ansprüche 4 und 5, bei dem die Sperrmittel (240; 242) ein Paar in einem Durchlaß in dem ersten Fortsatz angebrachte Keile (240; 242) und eine Stellschraube (246) enthält, um die Keile gegen das erste Paar von beabstandeten Wänden nach außen zu drängen.

7. Laryngoskophandgriff (160; 160a; 296; 330; 390; 500; 502) zur Verwendung in einem Laryngoskop, ausgelegt zum Betrieb mit einem ersten Spatel (310; 360; 397; 528) mit einem Lichtleiter (316; 376; 422; 478; 526), wobei der Handgriff enthält: einen Handgreifabschnitt, einen mit dem Handgreifabschnitt verbundenen und zur lösbaren Verbindung mit dem ersten Spatel ausgelegten Handgriffsverbindungs-Kopfabschnitt, Energiequellenmittel (162; 161a; 472; 510); eine erste Lichtquelle (180; 180a; 222; 284; 326; 350; 400; 432; 452; 516) und einen Teil des Kopfabschnittes bildende und mit dem Energiequellenmittel und der ersten Lichtquelle (180; 180a; 222; 284; 326; 350; 400; 432; 452; 516) verbundene Mittel (192; 190a; 230; 282; 324; 346; 408; 438; 456; 532; 557) zum Beaufschlagen der ersten Lichtquelle, wenn der erste Spatel lösbar mit dem Kopfabschnitt verbunden ist, dadurch gekennzeichnet, daß der Laryngoskophandgriff auch ausgelegt ist, mit einem zweiten Spatel (102; 340; 395) der eine eigene Lichtquelle (110; 336) besitzt, zusammenzuwirken; und daß einen Teil des Kopfabschnittes bildende und mit dem Energiequellenmittel (162; 161a; 472; 510) verbundene Mittel (174; 172; 206; 346; 396; 436; 462; 504) vorgesehen sind zur Beaufschlagung der durch den zweiten Spatel gehaltenen zweiten Lichtquelle (110; 136), wenn der zweite Spatel lösbar mit dem Kopfabschnitt verbunden ist, ohne die erste Lichtquelle (180; 180a; 222; 284; 326; 350; 400; 432; 452; 516) beaufschlagen zu lasen.

8. Laryngoskophandgriff nach Anspruch 7, in Kombination mit einem ersten Spatel (310; 360; 397; 528).

9. Laryngoskophandgriff nach Anspruch 7 in Kombination mit einem zweiten Spatel (102; 340; 395).

10. Laryngoskopspatel mit: einem Spatelkörper (528) mit einem vordersten und einem hintersten Ende, wobei das Letztere ein Befestigungs-Basisteil (540) definiert mit Mittelm zum Anbringen des Spatels an einem damit zusammenwirkenden Laryngoskophandgriff (500) in der Weise, daß eine spezifische Fläche des Basisteiles (538) in gegenüberliegender Beziehung mit einer damit zusammenwirkenden Fläche des Handgriffes ist; einem durch den Spatel gehaltenen und sich von dessen hintersten Ende an dem Basisteil zu seinem vordersten Ende an dem vordersten Ende des Spatelkörpers so erstreckenden Lichtleiter (526), daß das hinterste Ende des Lichtleiters in optischer Ausrichtung mit einer durch den Handgriff (500), wenn das Basisteil des Spatels an dem Handgriff angebracht ist, gehaltenen Lichtquelle (560) plaziert ist; elektrischem Kontaktmittel (537) an dem Basisteil, das dazu ausgelegt ist, mit einem damit zusammenwirkenden Kontaktmittel (532) an dem Handgriff in Eingriff zu treten, um die Lichtquelle (516) beaufschlagen zu lassen, wenn das Basisteil des Spatels lösbar mit dem Handgriff verbunden ist, dadurch gekennzeichnet, daß das elektrische Kontaktmittel (537) von der spezifischen Oberfläche (538) des Basisteils vorsteht und daß die spezifische Fläche des Basisteils des Spatellörpers eine Öffnung enthält, die gestaltet ist zur Aufnahme eines zweiten vorstehenden Kontaktmittels, das einen Teil des Handgriffes bildet, so daß das zweite Kontaktmittel nicht an dem Spatel angreift, wenn die Basis des Spatels lösbar mit dem Handgriff verbunden ist.

**Revendications**

1. Laryngoscope comprenant: une poignée (160; 160a; 296; 330; 390; 500; 502) comprenant une première source de lumière (180; 180a; 222; 284; 326; 350; 400; 432; 452; 516) et une source d'énergie (162; 161a; 472; 516); et une première lame (310; 360; 397; 528) portant un guide de lumière (316; 376; 422; 478; 526) mais aucune source de lumière et ayant des moyens pour le montage de la première lame avec ladite poignée d'une manière amenant ladite source d'énergie (162; 161a; 472; 510) à alimenter la première source de lumière (180; 180a; 222; 284; 326; 350; 400; 432; 452; 516) tout en plaçant ledit guide de lumière en communication optique avec ladite première source de lumière, caractérisé par la présence d'une seconde lame (102; 340; 395) portant une seconde source de lumière (110; 136); et par des moyens formant une partie de ladite poignée et une partie de ladite seconde lame pour le montage de la seconde lame (102; 340; 395) avec ladite poignée (160; 160a; 296; 330; 390; 500; 502) d'une manière qui amène ladite source d'énergie (162; 161a; 472; 510) à alimenter ladite seconde source de lumière (110; 136) sans alimenter ladite première source de lumière (180; 180a; 222; 284; 326; 350; 400; 432; 452; 516).

2. Laryngoscope selon la revendication 1, dans lequel ladite poignée comporte une extrémité de raccordement de lame supérieure ayant des premiers moyens de contact (192; 190a; 230; 282; 324; 346; 408; 438; 456; 532; 557) connectés électriquement à ladite source d'émergie (162; 161a; 472; 510); dans lequel ladite première lame comporte des moyens permettant son montage sur l'extrémité de raccordement de lame de ladite poignée d'une manière l'appliquant contre lesdits premiers moyens de contact (192; 190a; 230; 282; 324; 346; 408; 438; 456; 532; 557) afin d'amener ladite source d'énergie (162; 161a; 472; 510) à alimenter ladite source de lumière (180; 180a; 222; 284; 326; 350; 400; 432; 452; 516) portée par ladite poignée, dans lequel des deuxièmes moyens de contact (312; 540) sont prévus à ladite extrémité de raccordement de lame supérieure, lesdits deuxièmens moyens de contact étant isolés électriquement desits premiers moyens de contact et étant aussi connectés à ladite source d'énergie; et dans lequel ladite seconde lame (102; 340; 395) comporte des moyens permettant son montage sur l'extrémité de raccordement de lame de ladite poignée d'une manière la faisant porter contre lesdits deuxièmes moyens de contact sans qu'elle porte sur lesdits premiers moyens de contact afin d'amener ladite source d'énergie à alimenter ladite seconde source de lumière.

3. Laryngoscope selon la revendication 1, dans lequel ladite première lame (310; 528) présente une extrémité avant et une extrémité arrière, cette dernière définissant une embase de montage (312; 540); dans lequel lesdits moyens de montage de la première lame sur ladite poignée (296; 502) d'une manière amenant ladite source d'énergie (510) à alimenter ladite première source de lumière comprennent un premier agencement coopérant formant une partie de ladite poignée et une partie de ladite embase, ledit agencement coopérant comprenant un passage (320) dans ladite poignée, et des moyens de connexion électriques (324; 532) pour coupler ladite source de lumière à ladite source d'énergie, lesdits moyens de connexion électriques étant disposés à l'intérieur dudit passage; dans lequel lesdits moyens de connexion électriques ne s'étendent pas à l'extérieur dudit passage; dans lequel ledit agencement comprend aussi un élément en saillie (314; 537) porté par l'embase de ladite première lame afin de porter contre lesdits moyens de connexion électriques lorsque ladite première lame est raccordée de façon amovible à ladite poignée pour amener ladite source d'énergie à alimenter ladite première source de lumière, et dans lequel ladite poignée (296; 502) comprend un second agencement coopérant (504) séparé dudit agencement dernier cité afin de coopérer avec ladit seconde lame d'une manière telle que, lorsque ladite seconde lame peut être raccordée de façon amovible à ladite poignée, ledit second agencement amène la source d'énergie (510) située dans ladite poignée (296; 502) à activer la source de lumière portée par ladite seconde lame, sans cependant amener ladite source d'énergie à activer la source de lumière (326; 537) portée par ladite poignée.

4. Laryngoscope selon l'une quelconque des revendications précédentes, dans lequel la source de lumière (222; 284) incorporée dans ladite poignée est équipée d'un adaptateur (200; 260) monté sur une poignée de base pour coupler lesdites première et seconde lames à lasite poignée de base, dans lequel ladite poignée de base comporte une première paire de parois parallèles espacées définissant une première rainure et une première tige transversale s'étendant en travers de ladite première rainure; dans lequel ladite poignée de base est conçue pour recevoir une batterie définissant ladite source d'énergie, dans lequel ledit adaptateur présente une première extrémité pouvant être reliée à ladite poignée de base et une seconde extrémité pouvant être reliée aux embases desdites première et seconde lames; dans lequel ladite première extrémité comporte une saillie ayant deux parois latérales espacées conçues pour s'engager dans ladite première rainure, une gorge transversale (212; 268) conçue pour s'enclencher avec ladite première tige transversale, et des moyens de verrouillage (240; 242) destinés à verrouiller ladite saillie en position dans ladite première rainure; dans lequel ladite seconde extrémité comporte une seconde paire de parois parallèles espacées définissant une seconde rainure et une seconde tige transversale (206; 266) s'étendant en travers de ladite seconde rainure; dans lequel les embases desdites première et seconde lames comportent chacune une seconde saillie ayant deux parois latérales espacées conçues pour s'engager dans ladite seconde rainure et une gorge transversale conçue pour s'enclencher avec ladite seconde tige transversale (206; 266), des moyens d'encliquetage étant destinés à fixer de façon amovible lesdites embases à ladite seconde extrémité dudit adaptateur dans une position enclenchée; dans lequel ladite poignée comporte un premier contact électrique disposé à peu près centralement dans ladite première rainure, face à un second contact électrique coopérant (210; 272) prévu sur une partie de base de ladite première saillie; dans lequel ledit deuxième contact est connecté électriquement à un troisième contact (206) disposé à peu près centralement dans ladite seconde rainure; dans lequel ledit deuxième contact est connecté électriquement à ladite source de lumière (222; 284) prévue dans ledit adaptateur dans lequel un commutateur est prévu dans ledit adaptateur, ledit commutateur pouvant être actionné par ladite première lame dans ladite position d'enclenchement afin de fermer un circuit électrique entre ladite source de lumière (222; 284) et ladite batterie; dans lequel ladite seconde lame peut être substituée à ladite première lame, ladite seconde lame ayant un quatrième contact coopérant avec ledit troisième contact et un cinquième contact coopérant avec ladite seconde tige transversale pour fermer un circuit électrique vers ladite batterie afin d'alimenter la source de lumière de ladite seconde lame, ladite seconde lame étant réalisée de façon à ne pas actionner ledit commutateur; et dans lequel lesdites première et seconde tiges transversales sont connectées électriquement entre elles et à ladite batterie lorsque adaptateur est verrouillé dans une position dans ladite première rainure.

5. Laryngoscope selon la revendication 4, dans lequel ledit adaptateur (200) comporte une partie de corps en métal, assurant ainsi un contact électrique entre lesdites première et seconde tiges transversales.

6. Laryngoscope selon l'une des revendications précédentes 4 et 5, dans lequel lesdits moyens de verrouillage (240; 242) comprennent deux coins (240; 242) montés dans un passage situé dans ladite première saillie et une vis de blocage (246) destinée à appliquer à force verse l'extérieur lesdits coins contre ladite première paire de parois espacées.

7. Poignée de laryngoscope (160; 160a; 296; 330; 390; 500; 502) à utiliser dans un laryngoscope conçu pour travailler avec une première lame (310; 360; 397; 528) ayant un guide de lumière (316; 376; 422; 478; 526), ladite poignée comprenant: une partie de prise manuelle, une partie de tête de liaison de poignée reliée à ladite partie de prise manuelle et conçue pour être raccordée de façon amovible à ladite première lame; une source d'énergie (162; 161a; 472; 510); une première source de lumière (180; 180a; 222; 284; 326; 350; 400; 432; 452; 516) et des moyens (192; 190a; 230; 282; 324; 346; 408; 438; 456; 532; 557) formant une partie de ladite partie de tête et reliés à ladite source d'énergie et à ladite première source de lumière (180; 180a; 222; 284; 326; 350; 400; 432; 452; 516) pour alimenter cette dernière lorsque ladite première lame est raccordée de façon amovible à ladite partie de tête, caractérisée en ce que la poignée de laryngoscope est également conçue pour travailler avec une seconde lame (102; 340; 395) ayant sa propre seconde source de lumière (110; 136); et en ce que des moyens (174; 172; 206; 346; 396; 436; 462; 504) faisant partie de ladite partie de tête et reliés à ladite source d'énergie (162; 161a; 472; 510) sont prévus pour alimenter ladite seconde source de lumière (110; 136) portée par ladite seconde lame lorsque cette dernière est raccordée de manière amovible à ladite partie de tête sans provoquer l'alimentation de ladite première source de lumière (180; 180a; 222; 284; 326; 350; 400; 432; 452; 516).

8. Poignée de laryngoscope selon la revendication 7 en combinaison avec ladite première lame (310; 360; 397; 528).

9. Poignée de laryngoscope selon la revendication 7 en combinaison avec ladite seconde lame (102; 340; 395).

10. Lame de laryngoscope comprenant: un corps de lame (528) ayant une extrémité avant et une extrémité arrière, cette dernière définissant une embase de montage (540) ayant des moyens pour le montage de la lame sur une poignée coopérante (500) de laryngoscope afin qu'une surface spécifique de ladite embase (538) soit opposée à une surface coopérante de ladite poignée; un guide de lumière (526) porté par ladite lame est s'étendant de son extrémité arrière, située à ladite embase, jusqu'à son extrémité avant située à l'extrémité avant dudit corps de lame afin que l'extrémité arrière du guide de lumière soit placée en alignement optique avec une source de lumière (516) portée par ladite poignée (500) lorsque l'embase de ladite lame est montée sur ladite poignée; des moyens de contact électriques (537) situés sur ladite embase; lesdits moyens de contact étant conçus pour porter contre des moyens de contact coopérants (532) situés sur ladite poignée afin de provoquer l'alimentation de ladite source de lumière (516) lorsque l'embase de ladite lame est raccordée de façon amovible à ladite poignée, caractérisé en ce que lesdits moyens de contact électriques (537) font saillie de ladite surface spécifique (538) de ladite embase et en ce que ladite surface spécifique de l'embase dudit corps de lame présente une ouverture configurée pour recevoir des seconds moyens de contact en saillie faisant partie de ladite poignée afin que les seconds moyens de contact ne portent pas contre la lame lorsque l'embase de la lame est raccordée de façon amovible à la poignée.

FIG. I

FIG. 2

FIG. 3

FIG. 4

FIG. 5

FIG. 6

FIG. 7

FIG. 8

FIG. 9

FIG. IO

0 110 333

FIG. 11

FIG. 12

FIG. 13

FIG. 14

FIG. 15

FIG. 16

FIG. 17

FIG. 18

FIG. 19

FIG. 20

FIG. 21

310

316

312

318

314

324

326

322

320

296

**FIG. 22**

340

342

338 344 336 334

346 348

354 332

350 352

330

**FIG. 23**

FIG. 24

FIG. 25

FIG. 26

8

FIG. 27

FIG. 28

FIG. 29

FIG. 30　　　FIG. 31　　　FIG. 32

0 110 333

FIG. 33

FIG. 34

FIG. 35

FIG. 36

FIG. 37

FIG. 38

FIG. 39

FIG. 40

FIG. 41

FIG. 42

**FIG. 43**

540

535

537

526

528

**FIG. 44**

524

540

538

535

528

526

FIG. 45

FIG. 46

FIG. 47